# EUROPÄISCHE PATENTSCHRIFT

(11) **EP 0 488 171 B1**
(45) Veröffentlichungstag und Bekanntmachung des Hinweises auf die Patenterteilung: **15.05.1996**
(21) Anmeldenummer: 91120188.7
(22) Anmeldetag: 26.11.1991
(51) Int. Cl.: G01N 33/53, G01N 33/531

(54) **Waschlösung für festphasen-immunometrische Verfahren, die Stabilisatoren für das Markierungssystem enthält und ihre Verwendung**
Washing solution for solid phase immunometric methods, containing stabilisors for the labeling system and its application
Solution de lavage pour des méthodes immunométriques en phase solide, contenant des stabilisateurs pour le système marqué et son application

(30) Priorität: 28.11.1990 DE 4037764
(43) Veröffentlichungstag der Anmeldung: 03.06.1992
(73) Patentinhaber: BEHRINGWERKE Aktiengesellschaft, 35001 Marburg (DE)
(72) Erfinder: Dopatka, Hans-Detlef, Dr., W-3550 Marburg (DE)

(56) Entgegenhaltungen:
- EP-A- 0 196 518
- EP-A- 0 328 388
- EP-A- 0 431 682
- PATENT ABSTRACTS OF JAPAN vol. 14, no. 415 (P-1102)(4358) 7. September 1990

## Beschreibung

Die Erfindung betrifft eine Stabilisatoren für das Markierungsenzym enthaltende Waschlösung für festphasen-immunometrische Tests und die Verwendung dieser Waschlösung.

Festphasen-immunometrische Tests, beispielsweise der Enzym-Linked-Immunosorbent Assay (ELISA), erfordern bei der Durchführung einen oder mehrere Waschschritte. Hierbei wird die feste Phase mit der Waschlösung gespült, um unspezifisch angelagerte Substanzen, z. B. Immunglobuline oder überschüssige Reagenzien, z. B. ein Enzymkonjugat, zu entfernen. Geschieht dies in geeigneter Weise, ergibt sich als Testergebnis ein Meßsignal, das der Konzentration des nachgewiesenen Analyten entspricht. Weiterhin läßt sich dieses Ergebnis reproduzieren.

Enzymimmunoassays als solche sind dem Fachmann bekannt und in der Literatur beschrieben, siehe z. B. KYREIN, H.J., Ärztl.Lab. 24, 57 - 65 (1978). EP-A-0 196 518 beschreibt die Stabilisierung der Aktivität von Peroxidase oder Peroxidase-Konjugaten in Lösung.

Festphasen zur Verwendung in solchen Enzymimmunoassays sind ebenfalls dem Fachmann bekannt und in der Literatur beschrieben, siehe z. B. VOLLER, A. et al, Bull.World Health Organ. 53, 55 - 65 (1976).

Solche festphasen-immunometrische Tests können auch mittels Instrumenten abgearbeitet werden. Dabei werden die Waschschritte durch das Instrument durchgeführt. In diesen Systemen weisen die bekannten Waschlösungen, welche z. B. aus detergenzhaltigen Phosphatpuffern im Neutralbereich bestehen, bestimmte Nachteile auf. Bei der Verwendung solcher Instrumente zur Abarbeitung des Waschschrittes erreicht sowohl die Richtigkeit als auch die Reproduzierbarkeit des Meßsignals erst nach einiger Zeit, d. h. nach der Abarbeitung einiger Platten, ein akzeptierbares Maß (BURROWS, P.M. et al., J.Virol.Meth. 8, 207 - 216 (1984)).

Der vorliegenden Erfindung lag daher die Aufgabe zugrunde, eine Waschlösung zu finden, bei deren Verwendung in Instrumenten der ELISA auch bei sofortigem Gebrauch dieser Geräte korrekt abgearbeitet werden kann. Als Maß für die korrekte Testdurchführung gilt ein Meßsignal, das mit der Konzentration des nachgewiesenen Analyten korreliert sowie die Reproduzierbarkeit der erhaltenen Ergebnisse.

Instrumente im Sinne dieser Erfindung sind alle Instrumente, mit deren Hilfe Waschvorgänge in Enzymimmunoassays maschinell durchgeführt werden können, unabhängig davon, ob diese Instrumente in der Lage sind, weitere Schritte der Abarbeitung von ELISA Tests vorzunehmen.

überraschenderweise wurde nun gefunden, daß unabhängig von der Pufferbasis, dem pH oder anderen Zusätzen der Waschlösung der Zusatz von Stabilisatoren diese Aufgabe löst.

Stabilisatoren im Sinne dieser Erfindung sind Substanzen, die das Markierungsenzym stabilisieren, wie z. B. Tobramycin, Phenol und Phenolderivate, bevorzugt sind Phenole und Phenolderivate, die einen oder mehrere Substituenten tragen, die C₁-C₃-Alkyl, Chlor und/oder Brom sein können.

Generell können Enzyme auch durch Substrate und kompetitive Inhibitoren stabilisiert werden.

Gegenstand der Erfindung ist daher eine Waschlösung für heterogene Enzymimmunoassays, die einen Stabilisator für das Markerenzym enthält.

Gegenstand der Erfindung ist auch die Verwendung einer Waschlösung wie oben beschrieben in einem heterogenen Enzymimmunoassay.

Gegenstand der Erfindung sind ferner heterogene Enzymimmunoassays, wobei in mindestens einem Waschschritt eine Waschlösung wie oben beschrieben eingesetzt wird.

Gegenstand der Erfindung ist weiterhin die Verwendung von Stabilisatoren für das Markierungsenzym in Waschlösungen für heterogene Enzymimmunoassays.

Der Stabilisator wird in einer Konzentration von 0,01 bis 20 mM zugesetzt. Bevorzugt wird eine Konzentration von 0,1 bis 5mM, ganz besonders bevorzugt 1 mM. Der Stabilisator kann bereits bekannten Waschlösungen oder Puffern für festphasen-immunometrische Tests zugesetzt werden.

In einer bevorzugten Ausführung wird die Waschlösung gepuffert. Puffersysteme, die dafür verwendbar sind, sind dem Fachmann bekannt. Der im einzelnen verwendete pH hängt von dem Testsystem ab und kann gegebenenfalls durch Versuche ermittelt werden.

Bevorzugt als Stabilisatoren sind Phenole und Phenolderivate, wobei Phenol auch einen oder mehrere Substituenten tragen kann, die C₁-C₃-Alkylgruppen und Chlor und/oder Bromatome sein können.

Besonders bevorzugt sind die in den Beispielen dargelegten Lösungen, ganz besonders bevorzugt ist Phenol.

Heterogene Enzymimmunoassays sind an sich dem Fachmann bekannt. Sie können zum Nachweis von Antigenen und Antikörpern dienen und additiv, wie z. B. ein Sandwich-Immunoassay, oder kompetitiv sein. Die verschiedenen, möglichen Ausführungsformen sind hinlänglich in der Literatur beschrieben. Bevorzugt im Sinne der Erfindung ist das ELISA-Verfahren.

Markerenzyme für Enzymimmunoassays als solche sind literaturbekannt, bevorzugterweise werden alkalische Phospatase, β-Galaktosidase und Meerrettich-Peroxidase, besonders bevorzugterweise wird Merrettich-Peroxidase verwendet.

Festphasen für heterogene Enzymimmunoassays sind dem Fachmann an sich bekannt, bevorzugterweise werden konkave Formkörper, wie z. B. Röhrchen oder Näpfchen, konvexe Formkörper, wie z. B. Kugeln, Sterne o.ä. und Mikropartikel (Korngröße < 1000 nm) wie z. B. Latexpartikel und magnetisch anziehbare Partikel verwendet. Besonders bevorzugt sind dabei Näpfchen in Form von Mikrotitrationsplatten, Latexpartikel und magnetisch anziehbare Partikel.
Ganz besonders bevorzugt sind Mikrotitrationsplatten.

Materialien für Festphasen sind dem Fachmann bekannt. Soweit nicht, wie z. B. bei Latexpartikeln durch die Art der Festphase bereits festgelegt, wird bevorzugterweise Polystyrol verwendet.

Puffersysteme zur Verwendung in Enzymimmunoassays sind dem Fachmann bekannt. Dem Fachmann ist auch bekannt, daß die Art des jeweils verwendeten Puffersystems u. a. vom zu erzielenden pH abhängt.

Detergenzien zur Verwendung in Waschlösungen für heterogene Enzymimmunoassays sind dem Fachmann ebenfalls bekannt (siehe z. B. VOLLER, A. et al. Bull. World Health Organ. 53, 55 - 65 (1976)), bevorzugterweise verwendet werden dabei nichtionische und zwitterionische Detergenzien, besonders bevorzugt sind Polyoxyethylene, ganz besonders bevorzugt ist ^{R}Tween 20.

Neutralproteine zur Verwendung in Enzymimmunoassays sind dem Fachmann bekannt, bevorzugt verwendet werden z. B. Serumalbumine, Gelatine, chemisch modifizierte Gelatine, wie z. B. Polygelin, und Milchproteine, wie z. B. Lactoferrin, besonders bevorzugt sind Human- oder Rinderserumalbumin, Polygelin und Lactoferrin, ganz besonders bevorzugt sind Polygelin und Lactoferrin, letzteres hergestellt gemäß der Patentanmeldung DE 36 38 767.

Dem Fachmann ist bekannt, daß zur Einstellung einer definierten Osmolarität Neutralsalze, wie z. B. NaCl zu in Enzymimmunoassays verwendeten Lösungen hinzugegeben werden.

Die genannten Stoffe werden zum Gebrauch in wässriger Lösung verwendet; bis zum Gebrauch können sie z. B. in lyophilisierter oder granulierter Form vorliegen, als Trockenmischung oder in flüssiger Form, endverdünnt oder konzentriert.

Eine bevorzugte Ausführung der erfindungsgemäßen Waschlösung setzt sich wie folgt zusammen:

| | | |
|---|---|---|
| Puffer | 0...100 mmol/l, | bevorzugterweise 10-20 mmol/l ganz bevorzugterweise 10 mmol/l |
| Detergenz | 0 ... 1 %, | bevorzugterweise 0 ... 0,2 % ganz bevorzugterweise 0,1 % (w/v) |
| Neutralprotein | 0 ... 1 % (w/v) | |
| Stabilisator | 0,1...20 mmol/l, | bevorzugterweise 1 mmol/l |

Zusammensetzung und Bestandteile herkömmlicher Waschlösungen sind dem Fachmann bekannt.

Eine davon ist beispielsweise ein 10 mM Phosphatpuffer bestehend aus Na₂HPO₄, KH₂PO₄, NaCl 0,45 % w/v und 0,1 % (w/v) Tween^{R} 20 mit einem pH von 6,5. Diese Waschlösung wurde als Beispiel erfindungsgemäß mit 1 mM Phenol versetzt und diente dazu, die damit im ELISA erzielte Verbesserung des Meßsignals und der Reproduzierbarkeit zu belegen.

Das folgende Beispiel dient nur der Erläuterung und stellt in keiner Weise ein Einschränkung dar.

### Beispiel

Als Festphasen-immunometrischer Test wurde ein ELISA zum Nachweis von IgM gegen das humane Cytomegalovirus (CMV) gewählt. Als feste Phase dienten Mikrotitrationsplatten aus Polystyrene mit 96 Reaktionsvertiefungen im 8 x 12 Raster.

CMV, gezüchtet in Humanen Embryonalen Fibroblasten, und Humane Embryonale Fibroblasten, die nicht mit CMV infiziert waren, wurden nach Krishna et al (1980), J. Clin. Microbiol. 12, 46 - 51, zu Präparationen aufgearbeitet, die im weiteren als CMV-Antigen und als (negatives) Kontrollantigen bezeichnet werden.

Jede Mikrotitrationsplatte wurde nun durch Einpipettieren von 0,1 ml CMV-Antigen-Lösung in eine Reaktionsvertiefung und von 0,1 ml Kontrollantigen-Lösung in eine benachbarte Reaktionsvertiefung gemäß dem Verfahren der vorgenannten Autoren in der Weise beschichtet, daß Reihen von Reaktionsvertiefungen alternierend beschichtet für CMV-Antigen und Kontrollantigen entstanden. Mehrere Testplatten wurden auf diese Weise in demselben Produktionszyklus hergestellt.

Die Vorverdünnung und das Einpipettieren von je 0,15 ml der Untersuchungsproben erfolgte immer parallel in benachbarte Reaktionsvertiefungen, die einmal mit dem CMV-Antigen und zum anderen mit dem Kontrollantigen beschichtet waren gemäß den Anweisungen von Chou et al (1987), J. Clin. Microbiol. 25, 52 - 55.

Die Abarbeitung des ELISA folgte weitestgehend dem von Ziegelmaier et al (1981), J. Biol. Standard. 9, 23 - 33, beschriebenen Gang von Probeninkubation, Konjugatinkubation und Substratinkubation, wobei all diesen Reaktionsphasen ein Waschschritt vorausging.

### Testschema:

Beschichtung der Festphase mit viralem Antigen
*⁾ jeweils 3 x 200 µl Waschpuffer innerhalb von 5 min

### Waschschritt*⁾

- Schritt 1: 150 µl**⁾: Testserum in Verdünnungspuffer für Serum und Konjugat (VPSK) werden bei 37°C (für IgG und IgM) inkubiert

**⁾ Volumenangaben jeweils pro Vertiefung

### Waschschritt

- Schritt 2:: 50 µl anti-human IgG x AP Konjugat in VPSK, 60min bei 37°C

### Waschschritt

- Schritt 3:: 100µl p-Nitrophenylphosphat (p-NPP) in Substratpuffer, 45min bei 20-25°C
- Schritt 4:: 50µl 2N-NaOH

Optische Auswertung

Diese Waschschritte können nicht nur manuell mit einem Waschkamm, sondern auch automatisch mit einem Gerät durch geführt werden, das an ein Vorratsgefäß mit Waschlösung angeschlossen ist. Dem Stand der Technik entsprechen hier z. B. der Ultrawasher II von DYNATECH, der Microplatewasher von Flow Laboratories, der Immuno Washer NK 350 von NUNC, der Easy Washer "EAW plus" von SLT LABINSTRUMENTS oder der Behring ELISA Processor der Behringwerke. In vorgestelltem Beispiel wurde das zuletzt aufgeführte Gerät verwendet.

In Abänderung der Arbeitsweise von Ziegelmaier et al wurde nicht ein Anti-Human-IgG-Konjugat mit Alkalischer Phosphatase, sondern ein Anti-Human-IgM-Konjugat mit Peroxidase als Markerenzym benutzt. Als Substrat dieses Enzyms wurde Tetramethylbenzidin plus Wasserstoffperoxyd gewählt, welche gemäß den Patentanmeldungen DE 35 41 978 und DE 35 41 979 hergestellt worden waren. Die sich-entwickelnde Farbe ist nach 30 min mit 0,1 ml 0,5 N Schwefelsäure gestoppt und bei 450 nm in einem geeigneten Photometer, beispielsweise dem Gerät Titerek®, Multiskan MC, von Flow Laboratories oder dem Behring ELISA Processor der Behringwerke photometrisch vermessen worden. In vorgestelltem Beispiel wurde das zuletzt aufgeführte
Gerät verwendet.

Das mit der Untersuchungsprobe in der Kontrollantigenbeschichteten Reaktionsvertiefung erzielte Meßsignal wurde von dem von derselben Probe in der CMV-Antigen-beschichteten Reaktionsvertiefung erzielten Meßsignal subtrahiert. Die Differenz (ΔE) wird als spezifisches Signal (spez. O.D.) bezeichnet und ausschließlich bewertet.

Um den Einfluß der automatischen Waschlösungszugabe auf das ELISA-Ergebnis, nämlich spezifisches Signal sowie dessen Reproduzierbarkeit, zu verdeutlichen, wurde eine einfache Versuchsanordnung gewählt.

Eine Testplatte wurde in Mehrfachansätzen (n = 24) nur von zwei Untersuchungsproben belegt. Die Identifikation der einen Probe war PP 1635-3, die der anderen S 81-1 84cl. Vier weitere Testplatten wurden in identischer Probenbestückung angesetzt und der ELISA mit allen fünf Testplatten zusammen durchgeführt.

Hierbei wurde darauf geachtet, daß die Reihenfolge der einzelnen Testplatten, eins bis fünf, bei den anfallenden Waschschritten unverändert blieb.

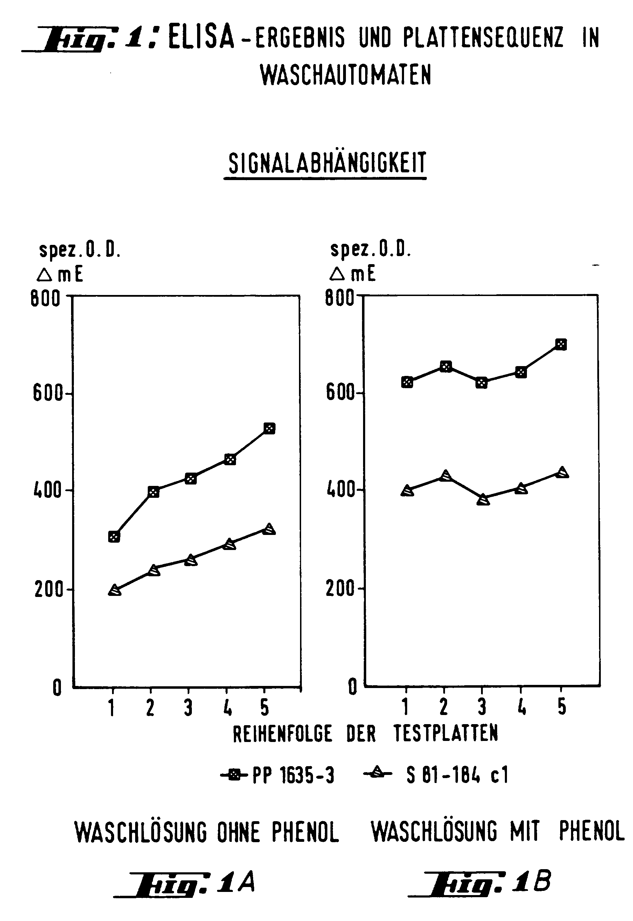

Zunächst wurde, ausgehend vom arithmetischen Mittelwert der spezifischen O.D., die Signalhöhe auf jeder Testplatte in Abhängigkeit von der Waschsequenz dargestellt (Fig. 1A). Eindeutig zeigte sich, daß die spezifische O.D. mit der Waschsequenz der Testplatten ansteigt.

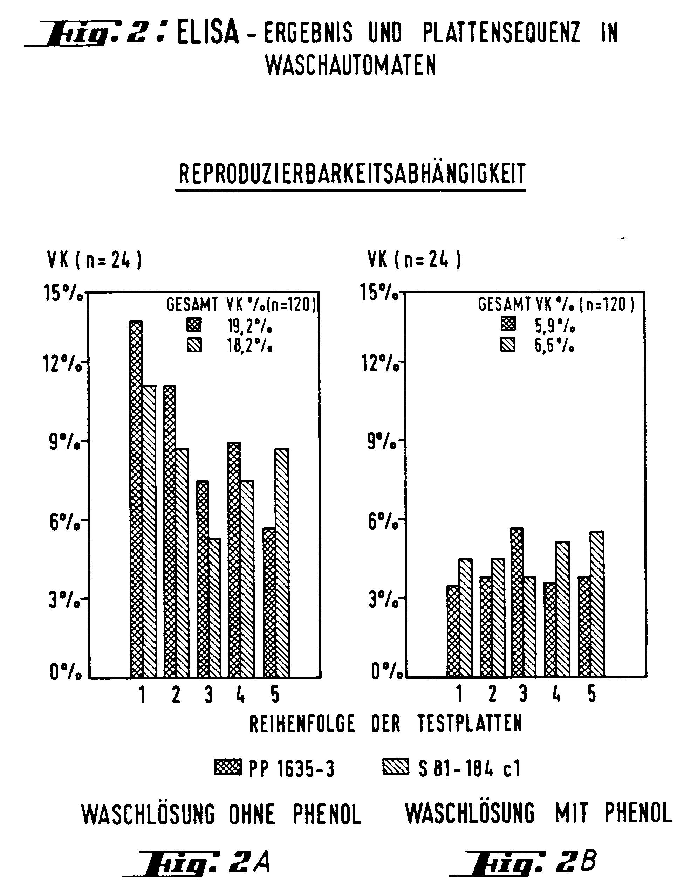

Dann wurde, ausgehend vom Variationskoeffizienten (VK) der spezifischen O.D., die Signalstreuung auf jeder Testplatte in Abhängigkeit von der Waschsequenz dargestellt (Fig. 2A). Eindeutig zeigte sich, daß der VK mit der Waschsequenz der Testplatten niedriger, d. h. besser wird.

Wird der identische Versuchsansatz in der Weise durchgeführt, daß der Waschlösung erfindungsgemäß Phenol, z. B. 0,1 mM, zugesetzt wird und diese Waschlösung mit einem Gerät in den Test eingebracht wird, so ergibt sich ein überraschend günstiges Ergebnis (Fig. 2B).

Die Mittelwerte der spezifischen O.D. liegen jetzt unabhängig von der Waschsequenz der Testplatten auf dem der Konzentration des CMV-spezifischen IgM entsprechenden Signalniveau (Fig. 1B). Daneben ist die Signalstreuung unabhängig von der Waschsequenz der Testplatte sehr gering.

Wird die auf sämtlichen fünf Testplatten erzielte Meßwertstreuung (Gesamt-VK) bewertet, ist die gemäß Erfindung erreichte Reproduzierbarkeitsverbesserung des ELISA-Ergebnisses noch drastischer. Bei einer Waschlösung nach dem Stand der Technik beträgt der Gesamt-VK je nach eingesetzter Untersuchungsprobe 18 bis 20 %. Bei einer Waschlösung mit dem erfindungsgemäßen Zusatz von Phenol und deren Anwendung beträgt der Gesamt-VK je nach eingesetzter Untersuchungsprobe 5 bis 7 %.

## Patentansprüche

1. Waschlösung zur Verwendung in heterogenen Enzymimmunoassays, dadurch gekennzeichnet, daß sie einen Stabilisator für das Markierungsenzym enthält.

2. Waschlösung nach Anspruch 1, dadurch gekennzeichnet, daß der Stabilisator in einer Konzentration zwischen 0,01 mM und 20 mM vorliegt.

3. Waschlösung gemäß Anspruch 1, dadurch gekennzeichnet, daß der Stabilisator ein Phenol oder ein Phenolderivat ist.

4. Waschlösung gemäß Anspruch 1, dadurch gekennzeichnet, daß der Stabilisator Phenol ist.

5. Waschlösung nach Anspruch 1, dadurch gekennzeichnet, daß das Phenol gegebenenfalls ein oder mehrere Substituenten trägt, die C₁-C₃-Alkylgruppen, Chlor- und/oder Bromatome sein können.

6. Waschlösung nach Anspruch 1, dadurch gekennzeichnet, daß sie einen Puffer enthält.

7. Waschlösung nach Anspruch 1, dadurch gekennzeichnet, daß sie ein Detergenz enthält.

8. Verwendung einer Waschlösung nach Anspruch 1 in einem heterogenen Enzymimmunoassay.

9. Verwendung gemäß Anspruch 8, dadurch gekennzeichnet, daß der Enzymimmunoassay ein ELISA ist.

10. Verwendung gemäß Anspruch 8, dadurch gekennzeichnet, daß die Waschschritte des Enzymimmunoassays mittels eines Gerätes durchgeführt werden.

11. Verwendung gemäß Anspruch 8, dadurch gekennzeichnet, daß das Markierungssystem Peroxidase ist.

12. Enzymimmunoassay, dadurch gekennzeichnet, daß in mindestens einem Waschschritt eine Waschlösung gemäß Anspruch 1 eingesetzt wird.

13. Verwendung von Stabilisatoren für das Markierungsenzym in Waschlösungen für heterogene Enzymimmunoassays.

## Claims

1. A washing solution for use in heterogeneous enzyme immunoassays, which contains a stabilizer for the labeling enzyme.

2. A washing solution as claimed in claim 1, wherein the stabilizer is present in a concentration between 0.01 mM and 20 mM.

3. A washing solution as claimed in claim 1, wherein the stabilizer is a phenol or a phenol derivative.

4. A washing solution as claimed in claim 1, wherein the stabilizer is phenol.

5. A washing solution as claimed in claim 1, wherein the phenol optionally carries one or more substituents which can be C₁-C₃-alkyl groups, chlorine and/or bromine atoms.

6. A washing solution as claimed in claim 1, which contains a buffer.

7. A washing solution as claimed in claim 1, which contains a detergent.

8. The use of a washing solution as claimed in claim 1 in a heterogeneous enzyme immunoassay.

9. The use as claimed in claim 8, wherein the enzyme immunoassay is an ELISA.

10. The use as claimed in claim 8, wherein the washing steps in the enzyme immunoassay are carried out by an apparatus.

11. The use as claimed in claim 8, wherein the labeling system is peroxidase.

12. An enzyme immunoassay, wherein a washing solution as claimed in claim 1 is employed in at least one washing step.

13. The use of stabilizers for the labeling enzyme in washing solutions for heterogeneous enzyme immunoassays.

## Revendications

1. Solution de lavage à utiliser dans les analyses immunoenzymatiques hétérogènes, caractérisée en ce qu'elle contient un stabilisateur pour l'enzyme marquée.

2. Solution de lavage selon la revendication 1, caractérisée en ce que le stabilisateur a une concentration comprise entre 0,01 mM et 20 mM.

3. Solution de lavage selon la revendication 1, caractérisée en ce que le stabilisateur est un phénol ou un dérivé phénolique.

4. Solution de lavage selon la revendication 1, caractérisée en ce que le stabilisateur est un phénol.

5. Solution de lavage selon la revendication 1, caractérisée en ce que le phénol, le cas échéant, porte un ou plusieurs substituants, qui peuvent être des groupes alkyle en C₁-C₃, des atomes de chlore et/ou de brome.

6. Solution de lavage selon la revendication 1, caractérisée en ce qu'elle contient un tampon.

7. Solution de lavage selon la revendication 1, caractérisée en ce qu'elle contient un détergent.

8. Utilisation d'une solution de lavage selon la revendication 1 dans une analyse immunoenzymatique hétérogène.

9. Utilisation selon la revendication 8, caractérisée en ce que l'analyse immunoenzymatique est un test ELISA.

10. Utilisation selon la revendication 8, caractérisée en ce que l'on effectue les étapes de lavage de l'analyse immunoenzymatique au moyen d'un appareil.

11. Utilisation selon la revendication 8, caractérisée en ce que le système marqué est une peroxydase.

12. Analyseimmunoenzymatique, caractérisée en ce que l'on utilise une solution de lavage selon la revendication 1 dans une étape de lavage au moins.

13. Utilisation de stabilisateurs pour le système marqué dans des solutions de lavage pour des analyses immunoenzymatiques.
